# EUROPÄISCHE PATENTSCHRIFT

(11) **EP 1 252 939 B1**
(45) Veröffentlichungstag und Bekanntmachung des Hinweises auf die Patenterteilung: **27.07.2005**
(21) Anmeldenummer: 02008936.3
(22) Anmeldetag: 22.04.2002
(51) Int. Cl.: B09B 1/00

(54) **Verfahren und Vorrichtung zur verbesserten Bewirtschaftung von Deponien**
Method and device for improved management of waste dumps
Procédé et dispositif d' exploitation améliorée de décharges

(30) Priorität: 25.04.2001 DE 10120107
(43) Veröffentlichungstag der Anmeldung: 30.10.2002
(73) Patentinhaber: Kress, Dieter, 91740 Röckingen (DE)
(72) Erfinder: Kress, Dieter, 91740 Röckingen (DE)
(74) Vertreter: Blaumeier, Jörg

(56) Entgegenhaltungen:
- DE-A- 3 921 949
- US-A- 5 201 609

## Beschreibung

Die Erfindung bezieht sich auf ein Verfahren zur verbesserten Bewirtschaftung von flüssigkeitsdicht abgedeckten Müll-Deponien mit verteilt angeordneten Gasbrunnen zur Abschöpfung des durch den biochemischen Abbauprozess entstehenden Deponiegases sowie eine Deponieanordnung zur Durchführung dieses Verfahrens.

Deponien werden seit geraumer Zeit großflächig abgedeckt, damit weder Oberflächenwasser, insbesondere Regenwasser, ständig in den Müllberg eindringt und dann kontaminiert als nur kompliziert und teuer zu entsorgendes Sickerwasser wieder austritt, sondern auch um unerwünschte Kontaminationen der Umgebung durch den Müllberg selbst zu vermeiden. Wenn eine solche Deponie oberflächig abgedeckt wird, so zeigt sich in kurzer Zeit, dass der biochemische Abbauprozess abklingt und folglich die Methangasproduktion versiegt. Die mit dem Müllabbau abgelagerten organischen Bestandteile können bei Wasserentzug nicht umgesetzt werden und mumifizieren. Tritt durch eine Undichtigkeit bei der technischen Deponieabdeckung in Zukunft wieder Oberflächenwasser in die Müllablagerung, so beginnt der Bioreaktor aufs neue seine Aktivität zu entwickeln. Dieser Neubeginn kann evtl. dann vonstatten gehen, wenn die sickerwasser- und deponiegasseitigen Entsorgungspfade nicht mehr funktionsfähig sind, so dass erst nachfolgende Generationen für eine geregelte Beseitigung Sorge tragen müssen.

Ein Ziel bei der Bewirtschaftung von Deponien ist es daher dafür zu sorgen, dass der biochemische Umsetzungsprozess in einer Zeit, in der die Entsorgungspfade (Gas und Sickerwasser) noch funktionsfähig sind, möglichst umfassend stattfindet, damit spätere Generationen durch die Deponie nicht mehr belastet sind.

In der Vergangenheit wurden auch in verschiedenen Deponien bereits Versuche durchgeführt, mit eingebohrten Metalllanzen bzw. Gräben oder Brunnen punktuell bzw. linear Sickerwasser in die Deponie zurückzuführen. Hierbei wurde festgestellt, dass infolge der Inhomogenität der Müllschüttung sich Wasserwegsamkeiten aufbauen, durch die ein direkter Kurzschluss zur Deponiebasis entsteht, worüber infiltriertes Wasser ohne den Befeuchtungseffekt wieder versickert. Darüber hinaus haben die vorgenannten Techniken bezogen auf ihre geringe Grundfläche gegenüber der grundsätzlich relativ großen Deponieoberfläche nur ein geringes Wirkungsspektrum.

Der Erfindung liegt daher die Aufgabe zugrunde, ein Verfahren zur verbesserten Bewirtschaftung von flüssigkeitsdicht abgedeckten Deponien zu schaffen, welches eine optimale Durchfeuchtung des Müllbergs im Sinne einer gezielten biochemischen Umsetzung der Inhaltsstoffe gewährleistet.

Zur Lösung dieser Aufgabe ist erfindungsgemäß vorgesehen, dass die in einzelne abgetrennte Versickerungsfelder unterteilte Deponieoberfläche in Abhängigkeit von der lokalen Gasergiebigkeit flächig von oben mit Wasser infiltriert wird, wobei das Infiltrationswasser, das vorzugsweise deponiespezifische Wässer, insbesondere das austretende Sickerwasser, umfassen kann, derart rasch aufgebracht wird, dass sich über das gesamte Versickerungsfeld ein freier Wasserspiegel aufstaut.

Durch das erfindungsgemäße Aufstauen des zur Anfeuchtung des Müllbergs im Bereich des jeweiligen Versickerungsfeldes dienenden Infiltrationswassers werden die eingangs beschriebenen Nachteile der punktuellen Wasserzuführung vermieden. Die gesamte Oberfläche des Versickerungsfeldes ist beispielsweise 30 cm hoch mit einer Wasserschicht, insbesondere Sickerwasserschicht, überdeckt, die dann langsam und großflächig in den darunter liegenden Müllberg einsinken kann. Dabei ergibt sich, dass eine - unabhängig von lokalen Dichteunterschieden im Müllberg - eine im wesentlichen gleichmäßige Durchfeuchtung des Müllbergs unterhalb des gefluteten Versickerungsfeldes erreicht wird. Dadurch ergeben sich optimale Bedingungen für die biochemische Umsetzung der abgelagerten organischen Bestandteile.

Zur Durchführung des erfindungsgemäßen Verfahrens ist eine Deponieanlage erfindungsgemäß dadurch gekennzeichnet, dass die Deponieoberfläche terrassiert und in durch flüssigkeitsdichte Trennschürzen voneinander getrennte Versickerungsfelder aufgeteilt ist, denen jeweils eine getrennt mit Infiltrationswasser beaufschlagbare Speiseleitung zugeordnet ist. Ein solches Versickerungssfeld kann eine Länge bis zu 60 und eine Breite von bis zu 10 m aufweisen, wobei die Abmessung zum einen von der Form und Größe des Müllbergs der Deponie abhängt, da ja die einzelnen Versickerungsfelder eine horizontale Oberfläche aufweisen müssen und zum anderen die Anordnung zweckmäßiger weise so getroffen sein sollte, dass in jedem Versickerungsfeld wenigstens ein Gasbrunnen angeordnet ist. Dies ermöglicht es durch die Auswertung des Gasvorkommens dieses Gasbrunnens eine Steuerung des Flutens des jeweiligen Versickerungsfeldes zu erreichen.

Die Trennschürzen können bevorzugt als schichtweise verdichtete Lehmrandwälle ausgebildet sein, die sich wenigstens 30 bis 50 cm über die planierte Oberfläche des Müllbergs im jeweiligen Versickerungsfeld erheben und darüber hinaus um etwa den gleichen Betrag auch noch unterhalb dieser planierten Oberfläche erstrecken.

In Ausgestaltung der Erfindung kann dabei vorgesehen sein, dass auf die planierte Oberfläche eines Versickerungsfeldes des Müllbergs eine vorzugsweise gleichkörnige Dränageschicht, beispielsweise aus Schotter oder Kies, aufgebracht ist, in der ein rundum gelochtes mit der Infiltrations-Speiseleitung verbundenes Drainagerohr verlegt ist. Auf die Drainageschicht wird ein Geotextil und darüber eine als Endabdichtung für die Deponie mit einer flüssigkeitsdichten Plane oder Abdichtung überdeckte Erdabdeckung zur Profilierung der Deponie aufgebracht. Diese Erdabdeckung zur Profilierung der Deponie ist wesentlich, damit Regenwasser ungehindert abfließen kann und nicht auf den für das Fluten mit Sickerwasser ja notwendigerweise ebenen Versickerungsfeldern stehen bleiben kann.

Um jederzeit einfach Reparaturen durchführen zu können und darüber hinaus auch Schwierigkeiten mit den im Betrieb unvermeidlichen Bewegungen und Setzungen des Müllbergs einer Deponie zu vermeiden, kann gemäß einem weiteren Merkmal der vorliegenden Erfindung vorgesehen sein, dass das Drainagerohr in einem Dosierschacht mit der von einer zentralen Verteilerstation gespeisten, den Lehmrandwall abgedichtet durchsetzenden Infiltrationsspeiseleitung verbunden ist, wobei wenigstens eines der Verbindungsenden von Speiseleitung und Drainagerohr mit einem Verschiebebewegungen ausgleichenden Kompensator versehen ist.

Die Speiseleitungen sollen dabei bevorzugt in der Erdabdeckung zur Profilierung der Mülldeponie-Oberfläche verlegt sein.

Zur Versorgung der einzelnen Versickerungsfelder mit dem Infiltrationswasser, insbesondere Deponie-Sickerwasser, - wobei im Falle unzureichender Mengen von Sickerwasser selbstverständlich Frischwasser oder insbesondere gesammeltes Oberflächenwasser zugesetzt werden kann, damit eine ausreichende Durchfeuchtung des Müllbergs stattfinden kann - kann in einer zentralen Verteilerstation ein Verteilerbalken für die Speiseleitungen zu den einzelnen Versickerungsfeldern und eine Steuereinheit zur Dosierung angeordnet sein, wobei der Verteilerbalken über eine Druckleitung mit einem Pumpwerk mit einem Infiltrationswasser-Speicherbecken verbunden ist. Dieses Speicherbecken ist dabei bevorzugt als Sickerwasserzwischenspeicher am tiefsten Punkt der Deponie angeordnet und mit der Sickerwasser-Ringdrainage der Deponie verbunden.

Wenn die Verteilerstation am höchsten Punkt der Deponie angeordnet ist, ergibt sich die besonders vorteilhafte Möglichkeit, die Speiseleitungen entleerbar zu machen, so dass sie getrennt an eine Saugleitung anschließbar sind, die mit Messgeräten für eine Gasanalyse und die Gasdurchflussmenge versehen ist. Man hat also auf diese Weise die Möglichkeit, unabhängig von der Erfassung des Methangases der einzelnen Gasbrunnen in der Gasregelstation eine zusätzliche Erfassung der Ergiebigkeit des jeweiligen Versickerungsfeldes vorzusehen, in dem die Speiseleitung eines zu untersuchenden Versickerungsfeldes entleert und an die Saugleitung angeschlossen wird, so dass das unter der Deponieabdeckung flächig aus der Oberfläche des Müllbergs in die Drainageschicht eindringende Zersetzungsgas in das Drainagerohr eingesaugt wird, so dass es sowohl auf seine Qualität hin analysiert als auch die anfallende Gasmenge gemessen werden kann. Diese anfallende Gasmenge kann dann wiederum dazu dienen festzulegen, wann und in welchem Umfang ein Fluten des entsprechenden Versickerungsfeldes zur Ankurbelung des biochemischen Zersetzungsprozesses im Müllberg unterhalb dieses Feldes stattfinden sollte.

Darüber hinaus kann selbstverständlich auch vorgesehen sein, dass die Steuereinheit im Verteilergebäude mit den Messeinrichtungen für die einzelnen Gasbrunnen in der Gasregelstation verbunden sind, so dass direkt aus der Ausbeute der einzelnen Gasbrunnen die notwendigen Informationen für das Fluten des diesen Gasbrunnen enthaltenden Versickerungsfeldes abgleitet werden.

Schließlich liegt es auch im Rahmen der Erfindung, die Absperrventile der einzelnen Speiseleitungen im Verteilergebäude programmgesteuert entsprechend der im jeweiligen Versickerungsfeld anfallenden Gasmenge zu betätigen, so dass letztendlich ein vollautomatischer Optimierungsbetrieb einer Deponie möglich ist.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: einen schematischen Lageplan einer erfindungsgemäß optimierten Deponie,

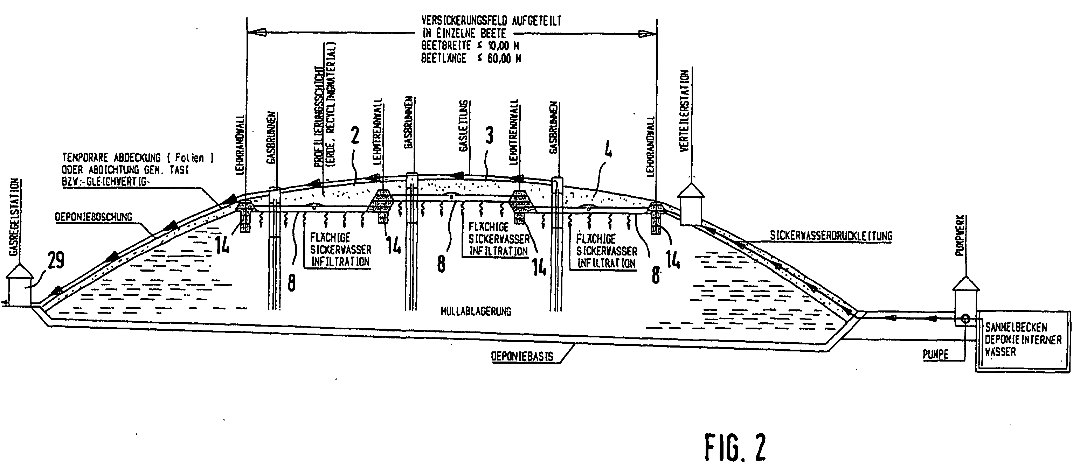

- Fig. 2: einen schematischen Schnitt durch die Deponie nach Fig. 1,

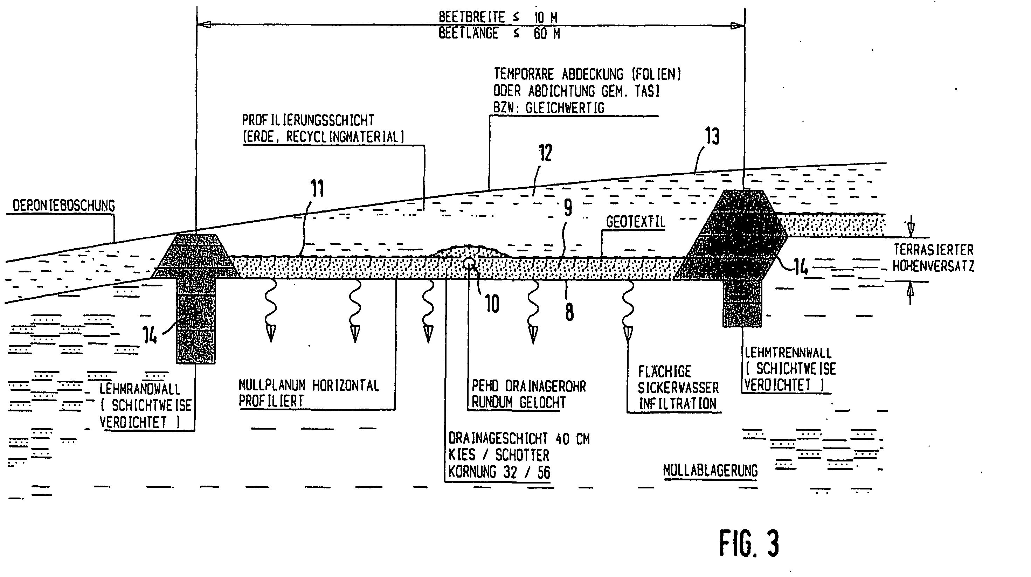

- Fig. 3: einen vergrößerten Querschnitt durch ein Versickerungsfeld der Deponie,
- Fig. 4: einen schematischen Teillängsschnitt durch ein Versickerungsfeld,
- Fig. 5: eine Schemazeichnung der Gasbrunnendurchdringung,
- Fig. 6: den Grundriss der Sickerwasserverteilerstation und
- Fig. 7: einen Schnitt A - A in Fig. 6

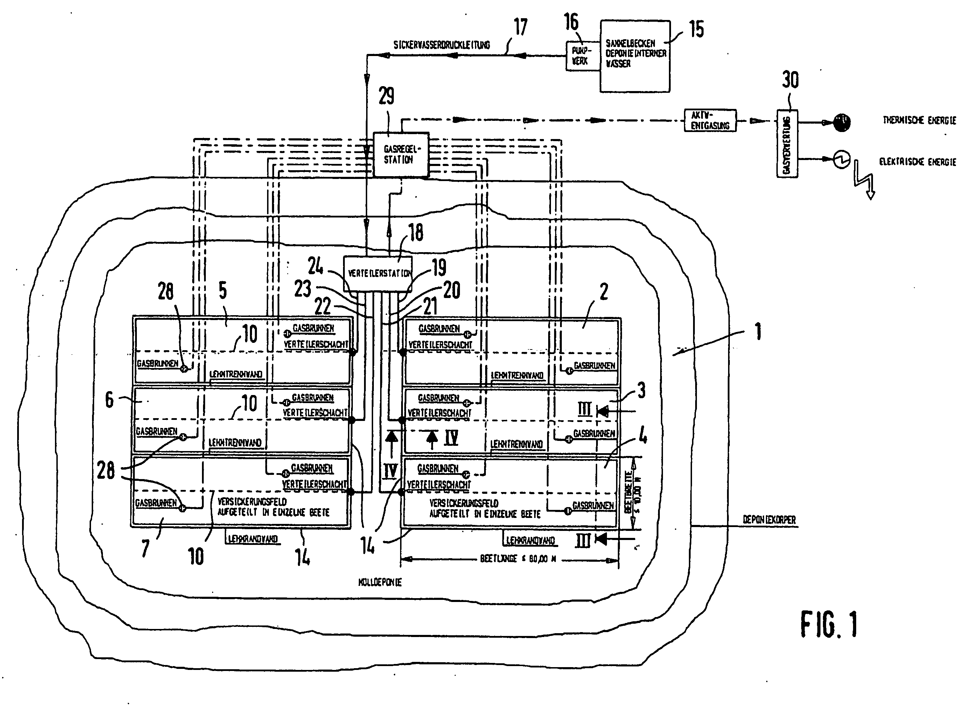

In Fig. 1 erkennt man bei 1 den mehr oder weniger terrassierten Deponiekörper, dessen Oberfläche erfindungsgemäß in einzelne Versickerungsfelder 2,3,4,5,6,7 aufgeteilt ist. Diese Aufteilung in sechs in Fig. 1 gezeigte Felder ist natürlich nur schematisch zu verstehen, da die Zahl und Größe sowie auch ggf. die Form dieser Versickerungsfelder der Oberfläche des Deponiekörpers angepasst wird. Bei Anlage einer neuen Deponie kann man hier einfacher entsprechende gleichartige Versickerungsfelder-Größen wählen. Bei einer nachträglichen Umrüstung einer Deponie auf das erfindungsgemäße Betreiberverfahren kann sich aber doch die Notwendigkeit ergeben, unterschiedliche Versickerungsfelder entsprechend den unterschiedlichen sinnvoll möglichen Terrassen bei der Terrassierung der Deponieoberfläche zu wählen. Auf die planierte Oberfläche 8 eines Versickerungsfeldes des Deponiekörpers 1 ist eine Drainageschicht vorzugsweise aus Kies/Schotter mit einer Körnung 32/56 und einer Dicke von etwa 40 cm aufgebracht, wobei in dieser Drainageschicht ein rundum gelochtes PEHD-Drainagerohr 10 verlegt ist, auf diese Drainageschicht 9 ist ein Geotextil 11 aufgebracht und auf dieses wiederum ist eine Profilierungsschicht 12, beispielsweise Erde, Recyclingmaterial oder dergleichen aufgebracht, um letztendlich eine gerundete Kuppe der Deponie zu erhalten, die entweder mit einer temporären Abdeckung 13 (also einer Folie) oder aber auch einer Abdichtung gemäß TASI aufgebracht sein kann. Durch die Profilierungsschicht ergibt sich eine Form bei der das Oberflächenwasser, also insbesondere das Regenwasser, gleichmäßig zu den unteren Rändern des Deponiekörpers ablaufen und dort gesammelt und abgeführt werden kann, ohne dass es in den Deponiekörper eindringt.

Die einzelnen Versickerungsfelder 2, 3 u. 4 sind durch schichtweise verdichtete Lehmrandwälle 14 voneinander getrennt, die bis über die Drainageschicht nach oben überstehen und sich auch ein ausreichendes Stück nach unten in den Deponiekörper erstrecken, so dass eine zumindest weitgehende Entkopplung der Versickerungsfelder und der unter ihnen liegende Bereiche des Deponiekörpers erreicht wird.

Durch Zuführen von Infiltrationswasser, insbesondere Sickerwasser von einem Sammelbecken 15 deponieinterner Wässer, über ein Pumpwerk 16, eine Sickerwasser-Druckleitung 17 und eine Verteilerstation 18 in einem zentralen Verteilergebäude können einzelne Sickerwasserspeiseleitungen 19,20,21,22,23 angesteuert werden, von denen jede zu einem Versickerungsfeld 2,3,4,5,6,7 und dem im wesentlichen mittig in diesem angeordneten Drainagerohr 10 führt.

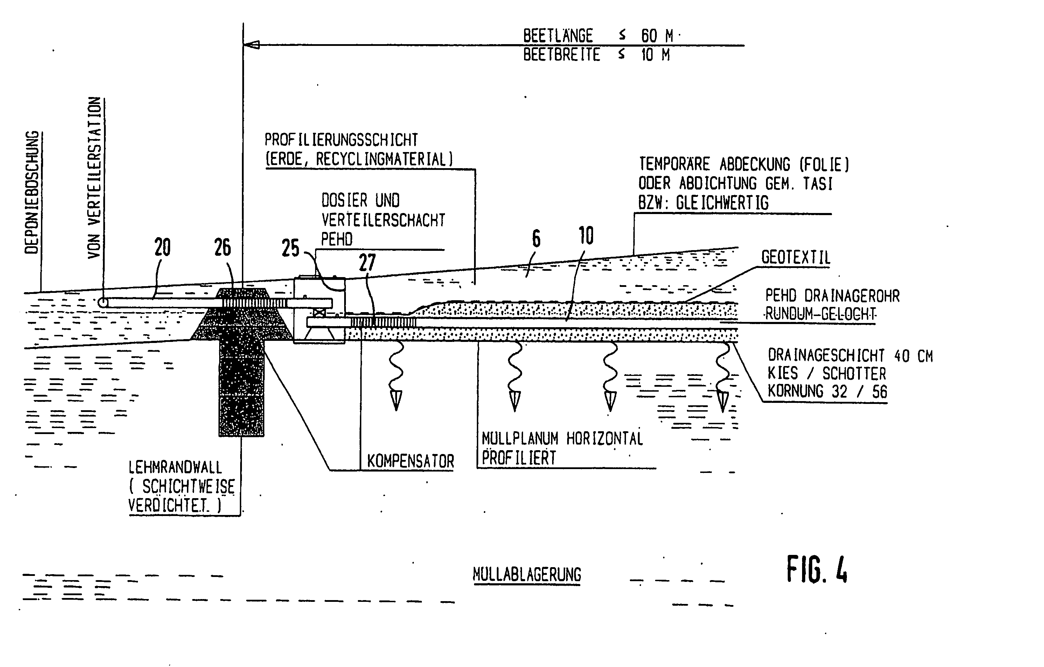

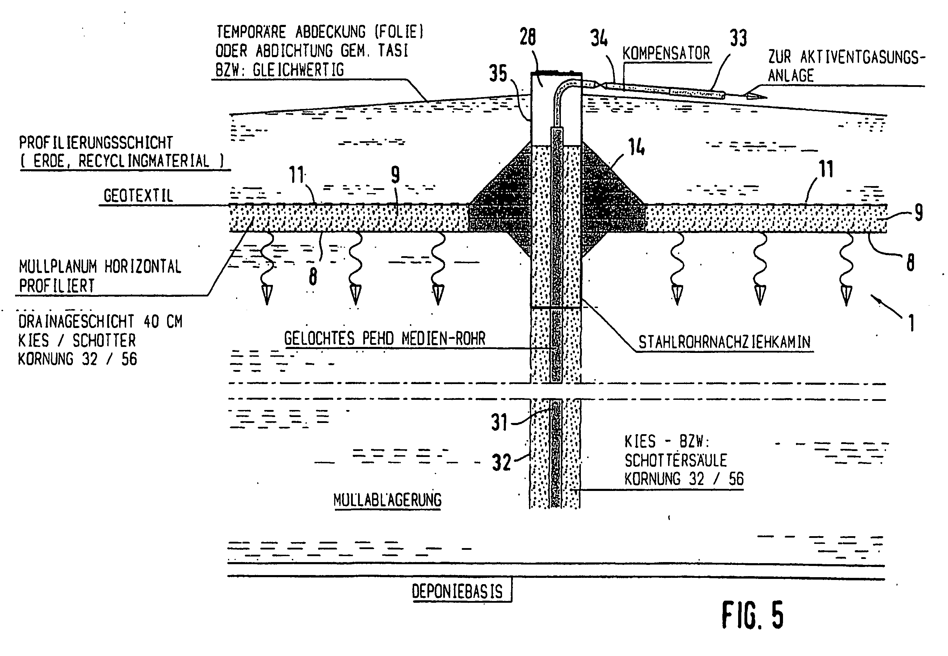

Die Speiseleitungen 19,20,21,22,23,24 sind, wie man insbesondere aus Fig. 4 erkennen kann, in einem Dosier- und Verteilerschacht 25 aus PEHD mit dem jeweiligen Drainagerohr 10 verbunden, wobei Kompensatoren 26 bzw. 27 dafür sorgen, dass die ständigen Bewegungen, insbesondere Setzbewegungen, in der Deponie nicht zu einem Brechen der Leitungen oder der Verbindung führen können. Bei 28 erkennt man Gasbrunnen, wobei solche handelsüblich ausgebildete Gasbrunnen bekannt sind, so dass sie nicht im Detail beschrieben zu werden brauchen. Je Versickerungsfeld sind etwa zwei Gasbrunnen vorhanden, die jeweils durch getrennte Leitungen mit einer Gasregelstation 29 verbunden sind, in der das Gas analysiert und der Gasverwertungsstation 30 zugeführt wird, wo entweder thermische Energie oder elektrische Energie erzeugt wird. Die Messgeräte der Gasregelstation sind mit der Steuereinheit in der Verteilerstation 18 verbunden, so dass entsprechend dem Gasaufkommen im jeweiligen Versickerungsfeld 2,3,4,5,6,7 ein Fluten dieses Feldes mit Sickerwasser stattfinden kann. Hierzu wird das Sickerwasser über die Drainageleitung 10 in einem solchen Ausmaß zugeführt, dass sich ein 30 cm hoher freier Wasserspiegel aufstaut. Das eingepumpte Sickerwasser hat dann Zeit, sich durch flächige druckfreie Versickerung im Müllkörper möglichst gleichmäßig auszubreiten und diesen zu infiltrieren. Schematisch ist ein solcher Gasbrunnen 28 in Fig. 5 dargestellt. Er umfasst ein gelochtes PEHD-Medienrohr 31, das in einem durch kies- bzw. schottergefüllten Bohrloch 32 des Deponiekörpers 1 angeordnet ist und über eine Gasleitung 32 mit Kompensator 34 mit der Gasregelstation 29 verbunden ist. Bei 32 erkennt man schematisch einen üblichen Stahlrohrnachziehkamin eines solchen Gasbrunnens.

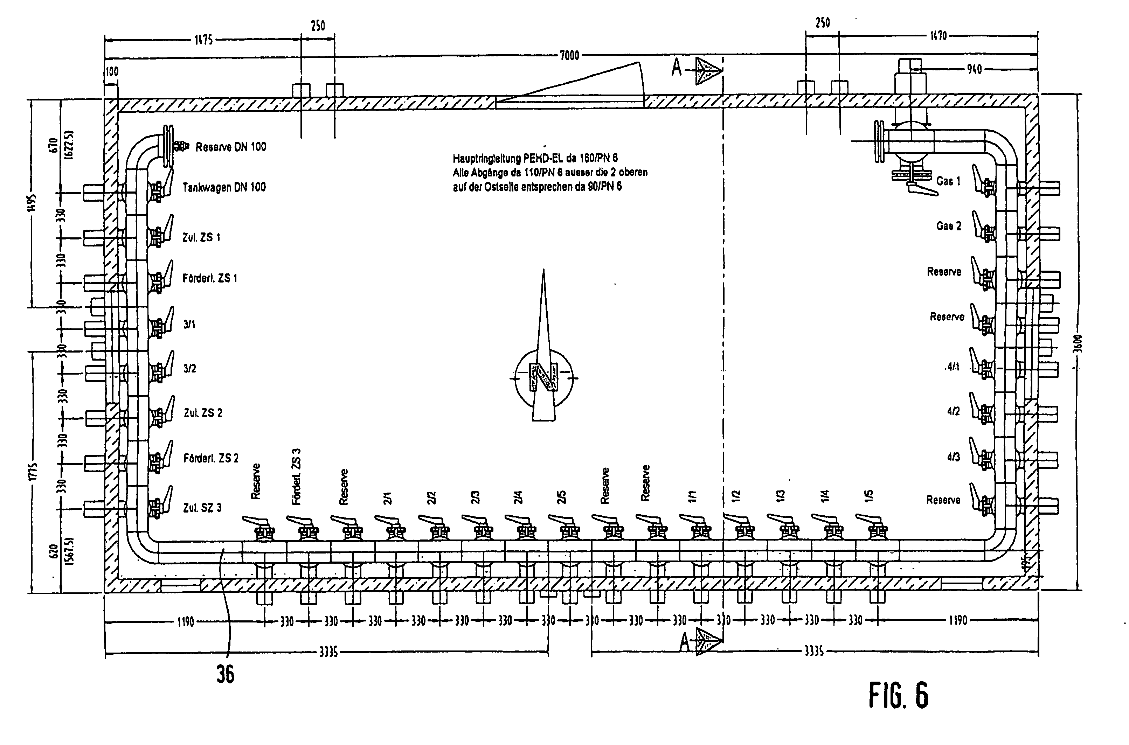

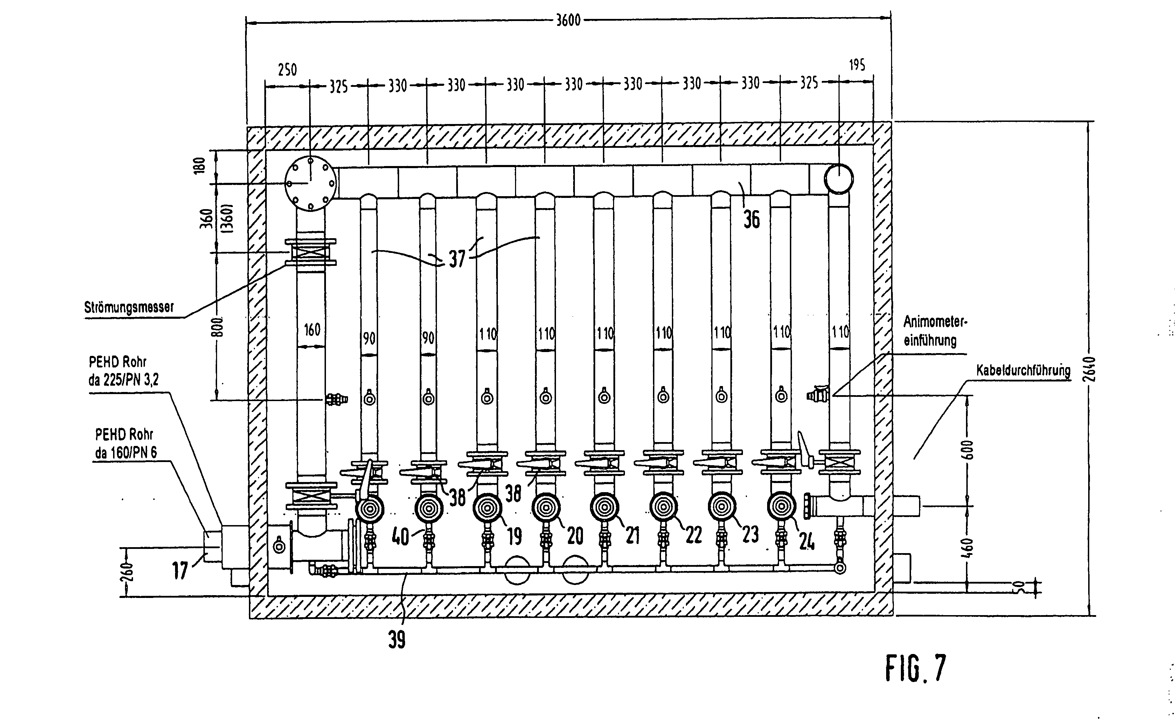

Der Aufbau der Verteilerstation 18 ist in den Fig. 6 und 7 gezeigt. Die Sickerwasserdruckleitung 17 wird auf einen Verteilerbalken 36 gelegt, von dem Stichleitungen 37 zu Verteilerventilen 38 führen, von denen jeweils eine Speiseleitung 19,20,21,22,23,24 abgeht. Diese Speiseleitungen können durch zu einer Sammelleitung 39 führende weitere Stichleitungen 40 entleert werden, so dass neben der Erfassung der Gasergiebigkeit eines Versickerungsfeldes 2,3,4,5,6,7 auch eine Gasmengenerfassung über die Drainagerohre 10 möglich ist. Nach dem Entleeren einer Speiseleitung 19,20,21,22,23,24 kann diese an eine Saugleitung angeschlossen werden, so dass über das Drainagerohr 10 im jeweiligen Versickerungsfeld 2,3,4,5,6,7 Gas angesaugt wird, das in diesem Versickerungsfeld großflächig nach oben austritt. Dieses Gas kann wiederum analysiert und vor allem seine Durchflussmenge bestimmt werden, aus der sich dann wieder in einem Rechnerprogramm bestimmen lässt, ob das jeweilige Versickerungsfeld neue geflutet werden muss, um eine stärkere Durchfeuchtung des Deponiekörpers unterhalb dieses Feldes und damit bessere biochemische Aktivität zu erhalten. Die Zahl der abgehenden Stichleitungen und auch der Speiseleitungen, die an den Veteilerbalken 36 der Sickerwasserverteilerstation anschließbar sind, ist in den Fig. 6 und 7 größer als die sechs, wie sie in der schematischen Fig. 1 eingezeichnet sind. Die sechs in Fig. 1 gezeigten Versickerungsfelder stellen ja ersichtlich nur einen Teil der Oberfläche des Deponiekörpers dar. In Wahrheit ergeben sich viel mehr Versickerungsfelder.

## Patentansprüche

1. Verfahren zur verbesserten Bewirtschaftung von flüssigkeitsdicht abgedeckten Deponien mit verteilt angeordneten Gasbrunnen zur Abschöpfung des durch den biochemischen Abbauprozess entstehenden Deponiegases, **dadurch gekennzeichnet, dass** die in einzelne abgetrennte Versickerungsfelder eingeteilte Deponieoberfläche in Abhängigkeit von der lokalen Gasergiebigkeit flächig von oben mit Wasser infiltriert wird.

2. Verfahren nach Anspruch 1, **dadurch gekennzeichnet, dass** zur Infiltration deponiespezifische Wässer, insbesondere austretendes Sickerwasser, verwendet wird.

3. Verfahren nach Anspruch 1 oder 2, **dadurch gekennzeichnet, dass** das Infiltrationswasser derart rasch aufgebracht wird, dass sich über das gesamte Versickerungsfeld ein freier Wasserspiegel aufstaut.

4. Deponieanlage zur Durchführung des Verfahrens nach einem der Ansprüche 1 bis 3, **dadurch gekennzeichnet, dass** die Deponieoberfläche in durch flüssigkeitsdichte Trennschürzen (14) voneinander getrennte Versickerungsfelder (2,3,4,5,6,7) aufgeteilt ist, denen jeweils eine getrennt mit Infiltrierwasser beaufschlagbare Speiseleitung (19,20,21,22,23,24) zugeordnet ist.

5. Deponieanlage nach Anspruch 4, **dadurch gekennzeichnet, dass** die Versickerungsfelder (2,3,4,5,6,7) eine Länge ≤ 60 m und eine Breite ≤ 10 m aufweisen.

6. Deponieanlage nach Anspruch 4 oder 5, **dadurch gekennzeichnet, dass** in jedem Versickerungsfeld (2,3,4,5,6,7) wenigstens ein Gasbrunnen (28) angeordnet ist.

7. Deponieanlage nach einem der Ansprüche 4 bis 6, **dadurch gekennzeichnet, dass** die Trennschürzen als Lehmrandwälle (14) ausgebildet sind.

8. Deponieanlage nach einem der Ansprüche 4 bis 7, **dadurch gekennzeichnet, dass** auf die planierte Oberfläche (8) eines Versickerungsfeldes (2,3,4,5,6,7) des Müllbergs eine vorzugsweise gleichkörnige Drainageschicht (19) aufgebracht ist, in der ein rundumgelochtes mit der Speiseleitung (19,20,21,22,23,24) verbundenes Drainagerohr (10) verlegt ist.

9. Deponieanlage nach Anspruch 8, **dadurch gekennzeichnet, dass** auf die Drainageschicht (9) ein Geotextil (11) und darüber eine mit einer flüssigkeitsdichten Plante oder Abdichtung (13) überdeckte Erdabdeckung (12) zur Profilierung der Deponie aufgebracht ist.

10. Deponieanlage nach einem der Ansprüche 4 bis 9, **dadurch gekennzeichnet, dass** das Drainagerohr (10) in einem Dosierschacht (25) mit der von einer zentralen Verteilerstation (18) gespeisten, den Lehmwall (14) abgedichtet durchsetzenden Infiltrations-Speiseleitung (19,20,21,22,23,24) verbunden ist.

11. Deponieanlage nach Anspruch 10, **dadurch gekennzeichnet, dass** wenigstens eines der Verbindungsenden von Speiseleitung (19,20,21,22,23,24) und Drainageroh (10) mit einem Verschiebebewegungen ausgleichenden Kompensator (26,27) versehen ist.

12. Deponieanlage nach einem der Ansprüche 4 bis 11, **dadurch gekennzeichnet, dass** die Speiseleitungen (19,20,21,22,23,24) in der Erdabdeckung (12) verlegt sind.

13. Deponieanlage nach einem der Ansprüche 10 bis 12, **dadurch gekennzeichnet, dass** in der zentralen Verteilerstation ein Verteilerbalken (36) für die Speiseleitungen (19,20,21,22,23,24) zu den einzelnen Versickerungsfeldern (2,3,4,5,6,7) und eine Steuereinheit zur Dosierung angeordnet ist, der über eine Druckleitung (17) und Pumpwerk (16) mit einem Infiltrationswasser-Speicherbecken (15) verbunden ist.

14. Deponieanlagen nach Anspruch 13, **dadurch gekennzeichnet, dass** das Speicherbecken (15) als Sickerwasserzwischenspeicher am tiefsten Punkt der Deponie angeordnet und mit der Sickerwasser-Ringdrainage der Deponie verbunden ist.

15. Deponieanlage nach einem der Ansprüche 10 bis 14, **dadurch gekennzeichnet, dass** die Verteilerstation (18) am höchsten Punkt der Deponie angeordnet ist.

16. Deponieanlage nach Anspruch 15, **dadurch gekennzeichnet, dass** die Speiseleitungen (19,20,21,22,23,24) entleerbar und getrennt an eine Saugleitung anschließbar sind, die mit Messgeräten für eine Gasanalyse und die Gasdurchflussmenge versehen ist.

17. Deponieanlage nach einem der Ansprüche 13 bis 16, **dadurch gekennzeichnet, dass** die Absperrventile (38) der einzelnen Speiseleitungen (19,20,21,22,23,24) in der Verteilerstation (18) programmgesteuert entsprechend der im jeweiligen Versickerungsfeld (2,3,4,5,6,7) anfallenden Gasmenge betätigbar sind.

18. Deponieanlage nach einem der Ansprüche 13 bis 17, **dadurch gekennzeichnet, dass** die Steuereinheit im Verteilergebäude mit den Messeinrichtungen für die einzelnen Gasbrunnen (28) in der Gasregelstation (29) verbunden sind.

## Claims

1. Method for the improved management of waste sites which are covered in a liquid-tight manner with distributed gas shafts for the extraction of the waste gas produced by the biochemical decomposition process, **characterised in that** the waste site surface divided into individually separate percolation areas is infiltrated with water from above as according to the local gas productivity.

2. Method according to claim 1, **characterised in that** for infiltration, waters specific to the waste site, in particular seepage water discharged therefrom, is used.

3. Method according to claim 1 or 2, **characterised in that** the infiltration water is applied sufficiently rapidly that a free water level builds up over the entire percolation area.

4. Waste site plant for carrying out the method according to one of claims 1 to 3, **characterised in that** the waste site surface is divided into percolation areas (2, 3, 4, 5, 6, 7) separated from one another by liquid-tight separating aprons (14), to each of which is allocated a supply line (19, 20, 21, 22, 23, 24) on which infiltration water may act separately.

5. Waste site plant according to claim 4, **characterised in that** the percolation areas (2, 3, 4, 5, 6, 7) have a length of ≤ 60 m and a width of ≤ 10 m.

6. Waste site plant according to claim 4 or 5, **characterised in that** in each percolation area (2, 3, 4, 5, 6, 7) at least one gas shaft (28) is provided.

7. Waste site plant according to one of claims 4 to 6, **characterised in that** the separating aprons are formed as clay peripheral walls (14).

8. Waste site plant according to one of claims 4 to 7, **characterised in that** on the levelled surface (8) of a percolation area (2, 3, 4, 5, 6, 7) of the mountain of refuse a drainage layer (19) preferably of equal grain size is applied, in which a drainage pipe (10) is laid, which is perforated all round and is connected to the supply line (19, 20, 21, 22, 23, 24).

9. Waste site plant according to claim 8, **characterised in that** on the drainage layer (9) a geotextile (11) and thereover a covering of earth (12) covered by a liquid-tight seal (13) is applied in order to shape the waste site.

10. Waste site plant according to one of claims 4 to 9, **characterised in that** the drainage pipe (10) is connected in a metering shaft (25) with an infiltration supply line (19, 20, 21, 22, 23, 24) which is supplied from a central distribution station (18) and which penetrates the clay wall (14) in a sealed manner.

11. Waste site plant according to claim 10, **characterised in that** at least one of the connecting ends of the supply line (19, 20, 21, 22, 23, 24) and drainage pipe (10) is provided with a compensator (26, 27) for compensating displacement movements.

12. Waste site plant according to one of claims 4 to 11, **characterised in that** the supply lines (19, 20, 21, 22, 23, 24) are laid in the earth covering (12).

13. Waste site plant according to one of claims 10 to 12, **characterised in that** in the central distribution station a distribution beam (36) is provided for the supply lines (19, 20, 21, 22, 23, 24) to the individual percolation areas (2, 3, 4, 5, 6, 7) and for a control unit for metering, which beam is connected via a pressure line (17) and pumping station (16) to an infiltration water reservoir (15).

14. Waste site plant according to claim 13, **characterised in that** the reservoir (15) is disposed as a seepage water intermediate reservoir at the lowest point in the waste site and is connected to the seepage water ring drainage of the waste site.

15. Waste site plant according to one of claims 10 to 14, **characterised in that** the distribution station (18) is disposed at the highest point of the waste site.

16. Waste site plant according to claim 15, **characterised in that** the supply lines (19, 20, 21, 22, 23, 24) may be evacuated and connected separately to a suction line, which is provided with measuring devices for analysing the gas and measuring the quantity of gas flowing through.

17. Waste site plant according to one of claims 13 to 16, **characterised in that** the locking valves (38) of the individual supply lines (19, 20, 21, 22, 23, 24) in the distribution station (18) may be actuated in a programme-controlled manner according to the quantity of gas produced in the respective percolation area (2, 3, 4, 5, 6, 7).

18. Waste site plant according to one of claims 13 to 17, **characterised in that** the control unit in the distribution building is connected to the measuring devices for the individual gas shafts (28) in the gas control station (29).

## Revendications

1. Procédé pour une exploitation améliorée des décharges à couverture imperméable aux liquides au moyen de sources de gaz disposées de façon subdivisée, en vue du prélèvement des gaz de décharge prenant naissance par le processus de dégradation biochimique, **caractérisé en ce que** la surface de la décharge, subdivisée en champs d'infiltration individuels séparés, est infiltrée par le haut par de l'eau, en fonction de l'abondance superficielle locale de gaz.

2. Procédé selon la revendication 1, **caractérisé en ce que**, en vue de l'infiltration, on utilise une eau spécifique à la décharge, en particulier des eaux d'infiltration sortantes.

3. Procédé selon l'une ou l'autre des revendications 1 ou 2, **caractérisé en ce que** l'eau d'infiltration est amenée rapidement d'une manière telle qu'elle s'accumule sur l'ensemble du champ d'infiltration sous forme d'une nappe d'eau libre.

4. Installation de décharge pour la mise en pratique du procédé selon l'une quelconque des revendications 1 à 3, **caractérisée en ce que** la surface de la décharge est subdivisée, par des tabliers séparateurs étanches aux liquides (14), en champs d'infiltration séparés les uns des autres (2, 3, 4, 5, 6, 7), à chacun desquels est associée une conduite d'alimentation séparée (19, 20, 21, 22, 23, 24) pouvant recevoir de l'eau d'infiltration.

5. Installation de décharge selon la revendication 4, **caractérisée en ce que** les champs d'infiltration (2, 3, 4, 5, 6, 7) présentent une longueur inférieure ou égale à 60 mètres et une largeur inférieure ou égale à 10 mètres.

6. Installation de décharge selon l'une ou l'autre des revendications 4 ou 5, **caractérisée en ce que**, dans chaque champ d'infiltration (2, 3, 4, 5, 6, 7), est installée au moins une source de gaz (28).

7. Installation de décharge selon l'une quelconque des revendications 4 à 6, **caractérisée en ce que** les tabliers séparateurs sont constitués sous forme de remblais en argile.

8. Installation de décharge selon l'une quelconque des revendications 4 à 7, **caractérisée en ce que**, sur la surface aplanie (8) d'un champ d'infiltration (2, 3, 4, 5, 6, 7) du tas de déchets est installée une couche de drainage (19), de préférence à grains uniformes, dans laquelle est placé un tuyau de drainage (10) à perforations périphériques rondes, relié à la conduite d'alimentation (19, 20, 21, 22, 23, 24).

9. Installation de décharge selon la revendication 8, **caractérisée en ce que**, sur la couche de drainage (9) est appliqué un géotextile (11), et, par dessus celui-ci, une protection en terre (12), recouverte de plantes ou d'un calfeutrage étanche aux liquides (13), en vue du profilage de la décharge.

10. Installation de décharge selon l'une quelconque des revendications 4 à 9, **caractérisée en ce que** le tuyau de drainage (10) est relié, dans une armoire de dosage (25), à une conduite d'alimentation par infiltration (19, 20, 21, 22, 23, 24) traversant de façon étanche le remblai en argile (14), et alimentée par une station centrale de répartition (18).

11. Installation de décharge selon la revendication 10, **caractérisée en ce qu'**au moins l'une des extrémités de liaison de la conduite d'alimentation (19, 20, 21, 22, 23, 24) et du tuyau de drainage (10) est équipée d'un compensateur (26, 27) équilibrant les déplacements lors du montage.

12. Installation de décharge selon l'une quelconque des revendications 4 à 11, **caractérisée en ce que** les conduites d'alimentation (19, 20, 21, 22, 23, 24) sont placées dans le recouvrement de terre (12).

13. Installation de décharge selon l'une quelconque des revendications 10 à 12, **caractérisée en ce que**, dans la station centrale de répartition, est installé un collecteur de répartition (36) des conduites d'alimentation (19, 20, 21, 22, 23, 24) allant vers les champs individuels d'infiltration (2, 3, 4, 5, 6, 7), et une unité de commande du dosage, ledit collecteur (36) étant relié, par une conduite sous pression (17) et un système de pompage (16) à un bassin de stockage d'eau d'infiltration (15).

14. Installation de décharge selon la revendication 13, **caractérisée en ce que** le bassin de stockage (15) est placé, en tant que moyen intermédiaire de stockage d'eau d'infiltration, au point le plus bas de la décharge, et est relié au système annulaire de drainage d'eau d'infiltration de la décharge.

15. Installation de décharge selon l'une quelconque des revendications 10 à 14, **caractérisée en ce que** la station de répartition (18) est placée au point le plus haut de la décharge.

16. Installation de décharge selon la revendication 15, **caractérisée en ce que** les conduites d'alimentation (19, 20, 21, 22, 23, 24) peuvent être séparément évacuées et raccordées à une conduite d'aspiration, qui est équipée de systèmes de mesure pour l'analyse de gaz et le débit de gaz.

17. Installation de décharge selon l'une quelconque des revendications 13 à 16, **caractérisée en ce que** les soupapes d'arrêt (38) des conduites d'alimentation individuelles (19, 20, 21, 22, 23, 24) peuvent être actionnées dans la station de répartition (18) selon un programme correspondant aux quantités de gaz se trouvant dans chacun des champs d'infiltration (2, 3, 4, 5, 6, 7).

18. Installation de décharge selon l'une quelconque des revendications 13 à 17, **caractérisée en ce que** les unités de commande se trouvant dans le bâtiment de répartition sont reliées avec les systèmes de mesure pour les sources individuelles de gaz (28) se trouvant dans la station régulatrice des gaz.
